(19)

Europäisches
Patentamt
European
Patent Office
Office européen
des brevets

(11)  **EP 2 112 902 B1**

(12)  **EUROPEAN PATENT SPECIFICATION**

(45) Date of publication and mention
of the grant of the patent:
**21.05.2014  Bulletin 2014/21**

(21) Application number: **05798789.3**

(22) Date of filing: **04.11.2005**

(51) Int Cl.:
**A61B 5/0225** *(2006.01)*     **A61B 5/021** *(2006.01)*
**G01L 17/00** *(2006.01)*     **G01L 11/02** *(2006.01)*

(86) International application number:
**PCT/SE2005/001657**

(87) International publication number:
**WO 2006/049571 (11.05.2006 Gazette 2006/19)**

(54) **PAD FOR MEASURING SYSTOLIC BLOOD PRESSURE IN THE ANKLE USING PPG**

PAD ZUR MESSUNG DES SYSTOLISCHEN BLUTDRUCKS IM FUSSGELENK MITTELS PPG

ELEMENT DESTINE A MESURER LA PRESSION SANGUINE SYSTOLIQUE DANS LA CHEVILLE
À L'AIDE DE PPG

(84) Designated Contracting States:
**AT BE BG CH CY CZ DE DK EE ES FI FR GB GR
HU IE IS IT LI LT LU LV MC NL PL PT RO SE SI
SK TR**

(30) Priority:  **04.11.2004  SE 0402673**

(43) Date of publication of application:
**04.11.2009  Bulletin 2009/45**

(73) Proprietor: **LB Index AB
587 31 Linköping (SE)**

(72) Inventors:
• **Lindberg, Lars-Göran
585 93 Linköping (SE)**
• **Jönsson, Björn
587 31 Linköping (SE)**

(74) Representative: **Thiel, Christian et al
Schneiders & Behrendt
Rechts- und Patentanwälte
Huestrasse 23
44787 Bochum (DE)**

(56) References cited:
**WO-A1-01/54575     WO-A1-97/49332
US-A- 4 821 734     US-B1- 6 533 729**

• **TANAKA S ET AL: "AMBULATORY INSTRUMENT
FOR MONITORING INDIRECT BEAT-TO-BEAT
BLOOD PRESSURE IN SUPERFICIAL
TEMPORAL ARTERY USING VOLUME-
COMPENSATION METHOD", MEDICAL AND
BIOLOGICAL ENGINEERING AND COMPUTING,
vol. 34, no. 6, 1 November 1996 (1996-11-01),
pages 441-447, XP000636569, ISSN: 0140-0118**
• **WHITELEY M S ET AL: "Photoplethysmography
can replace hand-held Doppler in the
measurement of ankle/brachial indices",
ANNALS OF THE ROYAL COLLEGE OF
SURGEONS OF ENGLAND, vol. 80, no. 2, March
1998 (1998-03), pages 96-98, XP002610014, ISSN:
0035-8843**
• **SADIQ S. AND CHITHRIKI M.: 'Arterial Pressure
Measurements Using Infrared Photosensors:
Comparison with CW Doppler' CLINICAL
PHYSIOLOGY, BLACKWELL SCIENTIFIC
PUBLICATIONS January 2001, OXFORD, GB,
pages 129 - 132, XP008020431**

EP 2 112 902 B1

**Description**

FIELD OF THE INVENTION

The present invention relates to a means for measuring systolic blood pressure in the ankle.

BACKGROUND OF THE INVENTION

The determination or monitoring of systolic blood pressure (SBP) is important for the appropriate control of patients with unstable haemodynamics. Invasive measurement by arterial catheters provides correct results but should be avoided, if possible, due to the risk thrombotic events and septicaemia, the formation of haematomae, and cost. Non-invasive methods for measuring or monitoring SBP utilise inflatable (pneumatic) cuffs by which the arterial blood flow in an arm, leg, or finger can be stopped. In the auscultatory or auditory method of Korotkov the pneumatic cuff is wrapped around an upper arm. By inflating the cuff the blood flow in the brachial artery is stopped. A stethoscope is placed over this artery distally off the cuff. On progressive deflation pulsating sounds are detected. SBP corresponds to the manometrically measured pressure in the cuff at which the pulsating sounds can be first heard while the diastolic blood pressure (DBP) corresponds to the lower pressure in the cuff at which the they disappear. Instead of the auscultatory method the Doppler effect of an ultrasound beach backscattered from moving red blood cells can be used for the measurement and monitoring of SBP. An ultrasound transceiver is placed over the artery distally of the cuff. The onset of arterial blood flow during deflation of the cuff corresponds to SBP, and the Doppler effect generated by the moving red blood cells gives rise to a sound signal in the audible range. Appropriate sites for measuring SBP by the Doppler method are the brachial and radial arteries of the arms and the tibial arteries of the legs. During monitoring the probe must be kept at a correct angle and a sufficient pressure against the skin. A third method for measuring SBP known in the art is the oscillometric method. The oscillating signal emanates from the small, pulse-dependent component in the cuff pneumatic pressure during standard (that is, auscultatory) blood pressure monitoring. The oscillating signal is caused by the mechanical coupling of the blood pressure in the artery which oscillates between SBP and consecutively lower pressure due to ongoing deflation of the cuff. The oscillometric signal is considered to have its maximum amplitude at a mean arterial blood pressure (MAP). From this signal oscillometric devices on the market calculate SBP and DBP using different algorithms most often kept secret by the respective manufacturer. SBP determined by oscillatory techniques is in good agreement with invasively obtained SPB (E O'Brien et al., Brit. Med. J. 322:531-536).

Determination of the systolic blood pressure in the ankle (ASP), and the calculation of the ankle-brachial blood pressure index (ABPI) are important elements in the investigation of leg ischemia. There is increasing interest in these parameters due to the association between a reduced ABPI and generalised arteriosclerotic disease.

Various methods for determining ASP are known in the art. In a standard method, a hand-held continuous-wave pen Doppler device of a frequency of about 8 to 10 MHz and a standard pneumatic cuff for temporarily restricting blood flow are used. Studies of this method (J Stoffers et al., Scand. J. Prim. Health Care 1991;9:109-114; K W Johnston et al., J. Vasc. Surg. 1987;6:147-151; F G R Fowkes et al., J. Epidem. Comm. Health 1988;42:128-133) indicated a variability in ABPI of 10% to 22% in a laboratory setting (95% confidence limitis for single measurement vs. mean of repeated measurements). In clinical practice the variability can be expected to be even higher due to intrinsic problems associated with this technique (S A Ray et al., Br. J. Surg. 1994;81:188-190; C M Fisher et al., J. Vasc. Surg. 1996;24:871-875). The examiner has to localise individual ankle arteries with the probe and keep it in the same position and angle during cuff inflation and deflation. Other devices for ASP measurement, such as automatic oscillometric pressure recorders (M Adiseshiah et al., Ann. Royal Coll. Surg. Engl. 1987;69:271-273; B Y Lee et al., J Vasc. Surg. 1996;23:116-122) are potentially less examiner-dependent. While giving comparable results to the Doppler method in healthy individuals, the oscillometric method is considered unreliable in arterial occlusive disease (B Jönsson et al., Clin. Physiol. 2001;21(2):155-163).

Photopletysmography (PPG) is a further technique by which SBP can be measured. A small area of the skin is illuminated by a light source directly or indirectly, such as via an optical fibre. The radiation, in particular red or infrared radiation, is diffusely scattered in the tissue and blood. A portion is scattered back and can be detected by a photo detector placed adjacent to the illuminated area (reflection mode). With thin tissues, such as on fingertips, it is also possible to detect the scatted light on a skin surface opposite to the illuminated area (transmission mode). PPG is used for non-invasively monitoring pulse rate, respiratory rate, tissue blood perfusion, arterial oxygen saturation, and blood pressure (K Yamakoshi et al., Med. Biol. Eng. Comput. 1982;20:307-313; R Chawla et al., Anesth. Analg. 1992; 74: 196-200; M Langbaum et al, J. Pediatr. 1994;125:591-595; U.S. Patent No. 6,120,459; The article by S. Tanaka and K. Yamakoshi, Med. & Biol. Eng. & Comput., 1996, vol. 34, p. 441-447, describes a disc-type cuff that may be attached to a user's head using a belt, for measuring blood pressure in the superficial temporal artery. The cuff includes a PPG sensor comprising two pairs of light emitting diodes and photo detectors, said pairs being disposed in parallel, the detectors being adapted for detecting light emitted by the respective diode into tissue and reflected from there, the cuff further comprising conductor means for providing power to the light emitting diodes from a power source and conducting means for putting the detectors in communication with electronic equipment for detector signal analysis.

US 6 533 729 B1 discloses a device for measuring SBP based on PPG. The sensor is mounted on a flexible band for application to the wrist, and comprises a plurality of light emitting diodes and photo detectors.

>

OBJECTS OF THE INVENTION

It is an object of the invention to provide a device for a non-invasive method for measuring systolic blood pressure in the ankle, which is simpler than methods known in the art.

It is another object of the invention to provide a device for a non-invasive method for measuring systolic blood pressure in the ankle, which is more reliable than methods known in the art.

Still further objects of the invention will become apparent from the following short description of the invention and a number of preferred embodiments thereof illustrated in a drawing, and the appended claims.

SHORT DESCRIPTION OF THE INVENTION

The invention relates to a PPG pad as detailed in claim 1.

The PPG pad of the present invention is useful in a method for measuring systolic blood pressure in the ankle of a subject, comprising

- providing an assembly including an ankle cuff, a flexible measuring pad ("PPG pad") comprising at least two pairs of light emitting diodes and photo detectors, and an electronic control unit in communication with the pad, the pad being optionally fixed at the cuff;
- positioning the pad or, if the pad is fixed at the cuff, the combination of pad and cuff in contact with the skin of the ankle region of the subject so as to dispose one pair in proximity of the anterior tibial artery and substantially parallel with it and the other pair in proximity of the posterior tibial artery and substantially parallel with it and, if the pad and cuff are separate, positioning the cuff around the pad that is in contact with the skin of the ankle region;
- inflating the cuff to a pressure sufficient for stopping blood flow through the anterior and posterior tibial arteries;
- deflating the cuff while making the diodes emit light;
- recording the light reflected from the tissue by the photo detectors during deflation;
- recording the cuff pressure during deflation;
- analysing the recorded light signal to identify the cuff pressure at which of blood flow in the anterior and/or posterior tibial arteries is resumed.

It is preferred to position the pad or the combination of pad and cuff on the ankle in a manner so as to dispose the photo detectors downstream of the light emitting diodes in respect of the blood flow in said arteries.

It is also preferred to position the pad in respect of the cuff so as to avoid compression of ankle tissue distally of the pad.

According to the present invention there is provided a flexible pad for measuring systolic ankle blood pressure (also termed "PPG pad"), comprising at least two pairs of light emitting diodes and photo detectors disposed in parallel, the detectors being adapted for detecting light emitted by the respective diode into tissue and reflected from there, the pad further comprising conductor means, preferably shielded, for providing power to the light emitting diodes from a power source and conducting means for putting the detectors in communication with electronic equipment for detector signal analysis. It is preferred for the pad to comprise a means for detector signal amplification, such as an AMP board. It is also preferred for the pad to be about rectangular in shape, its short sides extending in a proximal/distal direction when mounted. The pad is of a size that allows it to be wrapped around at least half of the circumference of the ankle of an adult person. It is also preferred for the SPMP to be divided into two sections of about rectangular shape, the line of division being parallel to the short sides, the sections being termed anterior tibial artery section and posterior tibial artery section. It is though within the ambit of the invention to position the light emitting diodes and/or the photo detectors elsewhere in/on the pad or separate of the pad while providing for light and/or optical signal communication by optical fibers extending from the positions at which the LED's and PD's are intended to be disposed according to the invention to the actual disposition of the LED's and PD's.

According to one preferred aspect of the invention, the light emitting diodes and the photo detectors of the pairs are disposed in a rectangular pattern or in a rectangular network pattern in the pad.

According to a second preferred aspect of the invention the at least two pairs of light emitting diodes and photodetectors are disposed in separate sections of the pad, each section comprising at least one such pair. In a pad with two such pairs, it is preferred for the distance between the light emitting diode and the photodetector in each pair to be smaller than the distance between the light emitting diodes and the photodetectors. In a pad with at least three such pairs, it is preferred for the distance between the light emitting diode and the photodetector in each pair to be smaller than the distance between the light emitting diodes and the photodetectors of the two adjacent pairs that pertain to separate sections.

According to a third preferred aspect of the invention, the pad or the combination of pair and cuff is provided with a mark indicating its correct position in regard of the *anterior margo tibiae*.

According to a fourth preferred aspect of the invention each light emitting diode and photodetector pair comprises an additional light emitting diode, preferably disposed in a linear manner on the cuff and with the photodetector positioned in-between the light emitting diodes.

According to a fifth preferred aspect, at least three pairs, each preferably comprising an additional photodetector disposed in the manner of the fourth preferred aspect, are arranged in each section of the pad.

According to a sixth preferred aspect a combination of the pad of the invention and an inflatable ankle cuff is provided. Preferably, the pad is fixed to the cuff, either permanently or, more preferred, releasingly, such as by Velcro® means.

According to the present invention is also provided a system for measuring systolic blood pressure in the ankle of a subject, comprising the measuring pad of the invention, an ankle cuff that may be fixed to the pad or be separate, pump means for inflating and deflating the cuff, gauge means for recording the pressure in the cuff, electronic means for amplification of photodetector signals, computing means for analysing the amplified signal, and power supply means.

The invention will now be explained in greater detail by reference to the drawings.

DESCRIPTION OF THE FIGURES

Fig. 1 is a rough sketch of a transverse section through a leg at its plane of smallest circumference at which the PPG pad is mounted;

Fig. 2 is a preferred embodiment of the PPG pad of the invention, in a top view of its application face;

Fig. 3 is a transverse section along line A-A through the PPG pad of Fig. 2 in combination with a inflatable cuff;

Fig. 4 is the PPG pad of Fig. 2, disposed on the cuff, in the same view as in Fig. 2;

Fig. 5 is a schematic view of a preferred embodiment of the system of the invention for measuring ankle systolic blood pressure;

Fig. 6 is a scheme illustrating the circuitry of the embodiment of Fig. 5;

Fig. 7 is a diagram showing raw PPG pad sensor signal amplitude v. cuff pressure traces for the anterior tibial artery and the posterior tibial artery during deflation obtained with the system of Fig. 5;

Fig. 8 is a diagram showing the amplitude v. cuff pressure traces refined by computer analysis of the corresponding traces of Fig. 7, the identification of the first true pulsatile signal in the anterior tibial artery (ATA 1) and the posterior tibial artery (PTA 1), and the corresponding cuff pressure;

Fig. 9 is a graph comparing systolic ankle pressure data obtained by the method disclosed herein and from state-of-the-art CW Doppler measurements;

Fig. 10 is a graph illustrating the effect of incorrect positioning of the PPG pad of the invention on PPG measurements of systolic blood pressure in the ankle; and

Fig. 11 is a graph comparing systolic blood pressure in the ankle obtained by the method disclosed herein and by an invasive method.

DESCRIPTION OF PREFERRED EMBODIMENTS

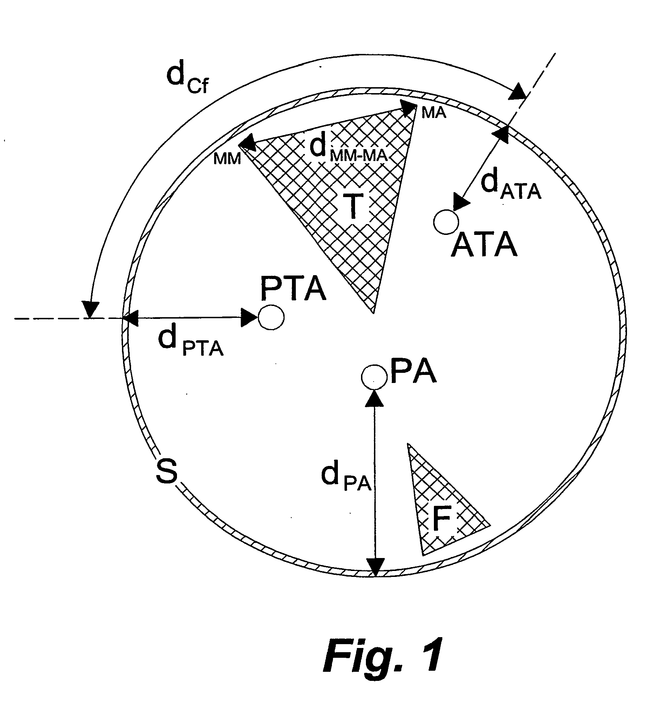

EXAMPLE 1. Anatomic considerations. Fig. 1 illustrates schematically a transverse section through a right leg at its plane of smallest circumference at which the PPG pad is mounted, with tibia T, fibula F, anterior tibial artery A, posterior tibial artery PTA, peroneal artery PA, and skin S shown.

Leg circumference at its most narrow ankle section was measured in 84 individuals (Table 1; age 18-85 years, median 67 years; 52 men, 32 women; 66 patients, 18 healthy volunteers; both legs).

**Table 1. Leg circumference and distance of arteries and their cutaneous projections in an ankle cross sectional plane**

|  | Range | Mean | SD |
|---|---|---|---|
| *Leg circumference (161 legs in 84 subjects*[*]), mm Distance artery/cutaneous proj.* (both legs in 20 subjects), mm | 170 - 265 | 218 | 20 |
| - Anterior tibial a. | 9 - 26 | 15 | 4 |
| - Posterior tibial a. | 8 - 24 | 15 | 4 |
| - Peroneal a. | 13 - 36 | 22 | 5 |

[*]) Legs with ankle edema (n=7) excluded

In 20 of the legs the distance between the skin surface and the major ankle arteries, $d_{ATA}$ for the anterior tibial artery, $d_{PTA}$ for the posterior tibial artery, $d_{PA}$ for the peroneal artery, was measured by colour-coded Duplex ultrasound (Table 1; ATL HDI5000 with an L 7-4 linear probe, Philips, Netherlands). In addition, the distance $d_{cf}$ between the cutaneous (skin designated S in Fig. 1) projections of the anterior ATA and posterior PTA tibial arteries in the cross sectional plane was measured in the right leg of 34 other subjects of the study group (19 patients, 15 healthy volunteers) by 8 MHz or 10 MHz hand-held Doppler ultrasound probes (Table 1). The width of the tibial bone in the same plane, measured as the distance $d_{MM-MA}$, between the *margo anterior* MA, the easily palpable frontal edge of the tibia, and the *margo medialis* MM, the lateral (inward) edge of the tibia, as calculated from nuclear magnetic resonance tomography (MR) images of one leg in seven healthy volunteers (26-52 years; five men, two women). A mean of 25 mm (SD 2, range 23-28 mm was obtained for $d_{MM-MA}$.

EXAMPLE 2. PPG pad design. Based on the anatomical findings, the features of a probe for measuring systolic blood pressure at the ankle were set to allow a variation of more than two standard deviations from the mean values in ankle circumference and in the distance between the arteries. The thickness of the tibial bone and its relation to the arteries, based on the MR scans, were also taken into account in setting the distance between the two channels. By the design of the probe, the considerably deeper located peroneal artery was assumed to be essentially beyond the detection range.

The positioning of the probe pays regard to the asymmetry of the arteries relative to the easily palpable frontal edge of the tibia. A marker on the probe (or one outside the cuff if a fixed probe/cuff combination is used) is positioned in line with this anatomical landmark. Since the anatomy of the right ankle is a reflection of the left and the probe prototype is asymmetrical, it has to be rotated 180° when right and left legs are examined, respectively.

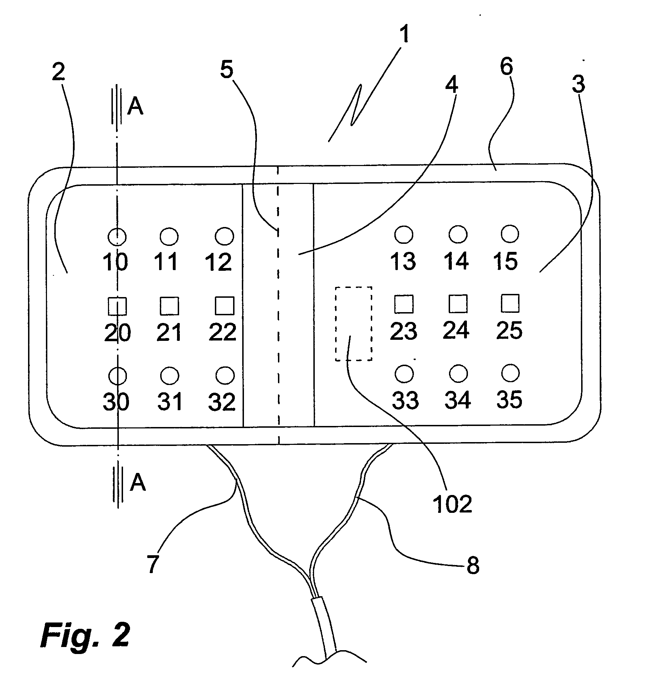

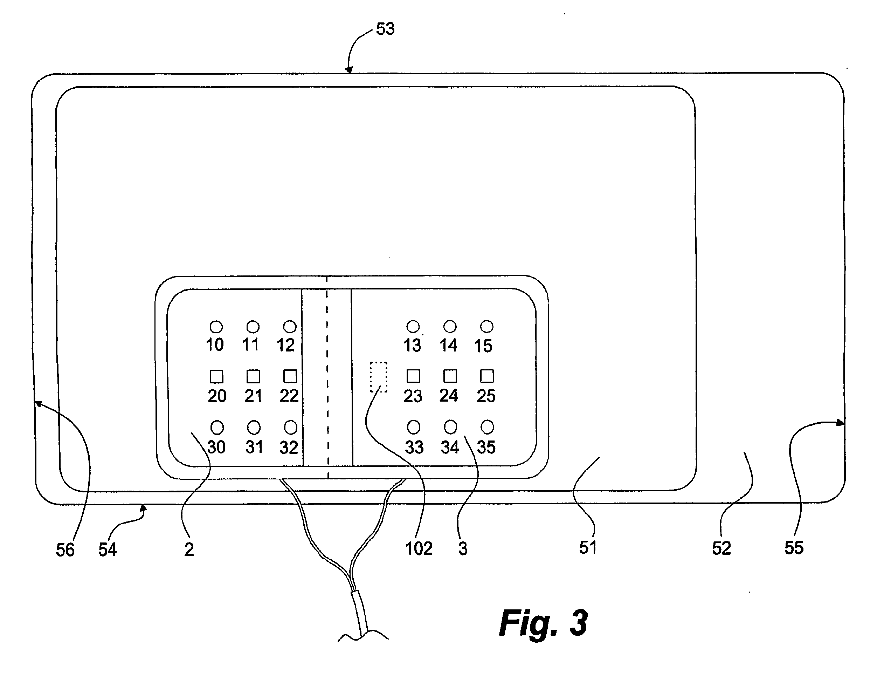

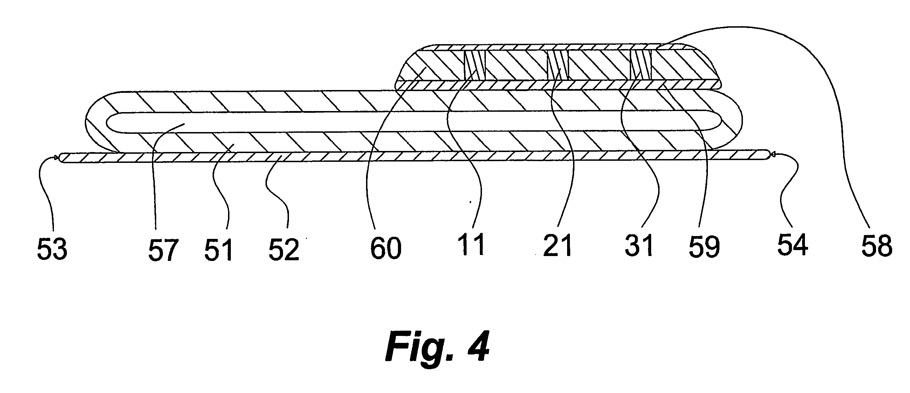

The about rectangular flexible PPG pad 1 according to a preferred embodiment of the invention illustrated in Figs. 2 and 3 is shown in a disposition for application to the left ankle of a person. The hatched line 5 indicates how the pad 1 is aligned with the *margo anterior tibiae;* a corresponding mark is provided at the rear (outer, when applied to the ankle) face of the pad 1 or the cuff, if a fixed combination of pad 1 and a cuff 50 (Fig 4) is used. When the pad 1 is applied to the left ankle, the hatched line 5 will abut the skin at the front of the left leg in close proximity of the tibia, with which it is aligned. The pad 1 comprises a sheet of soft, flexible silicone material 2, 3, 4, 6 in which a number of first 10, 11, 12, 13, 14, 15 and second 30, 31, 32, 33, 34, 35 light emitting diodes (LED) and photo detectors (PD) 20, 21, 22, 23, 24, 25 are embedded in three rows perpendicular to the hatched line 5, two LED rows and one PD row. Additionally embedded is an AMP board 102 for photodetector signal amplification. When applied to the subject the portion of the pad 1 on the top of Fig. 2 becomes the proximal portion; correspondingly the portion at the bottom becomes the distal portion. Half of the LEDs in the first or proximal LED row 10, 11, 12, 13, 14, 15 and the LEDs in the second or distal LED row 30, 31, 32, 33, 34, 35 are disposed on either side of line 5. Thus LEDs 10, 11, 12 of the first, proximal LED row and LEDs 30, 31, 32 of the second, distal LED row become disposed in proximity of the anterior tibial artery ATA; they are termed ATA LEDs. The other half of the LEDs (first or proximal LED row: 13, 14, 15; second or distal LED row: 33, 34, 35) become disposed in proximity of the posterior tibial artery PTA) they are termed PTA LEDs. Similarly, half of the photo detectors 20, 21, 22, 23, 24, 25 are arranged to the left of line 5, in which disposition they will primarily detect light reflected from the anterior tibial artery area; consequently they are termed ATA PDs 20, 21, 22. The other half of photo detectors 20, 21, 22, 23, 24, 25 are arranged to the right of line 5, in which disposition they will primarily detect light reflected from the posterior tibial artery area; consequently they are termed PTA PDs 23, 24, 25. The left and right (in relation to line 5) PPDs and photo detectors define left (anterior) and right (posterior) pad sections 2 and 3, respectively, which are separated by an intermediate section 4 that may be of a thinner flexible material such as, for instance, a textile material. Sections 2, 3, 4 are surrounded by a narrow circumferential border 6. The LEDs and PDs in the left and right portions of a row are disposed in an equidistant manner, the distance between them being about 15 mm. The distance of the innermost members (those closest to line 5) in a row is about 50 mm but the distance of the innermost member of a left row from line 5 is substantially shorter (about 15 mm) that the corresponding distance of the innermost member of a left row (about 35 mm); this reflects about the disposition of the corresponding arteries in relation to the front edge (or line 5) of the tibia. The distance between rows in a proximal/distal direction is about 20 mm.

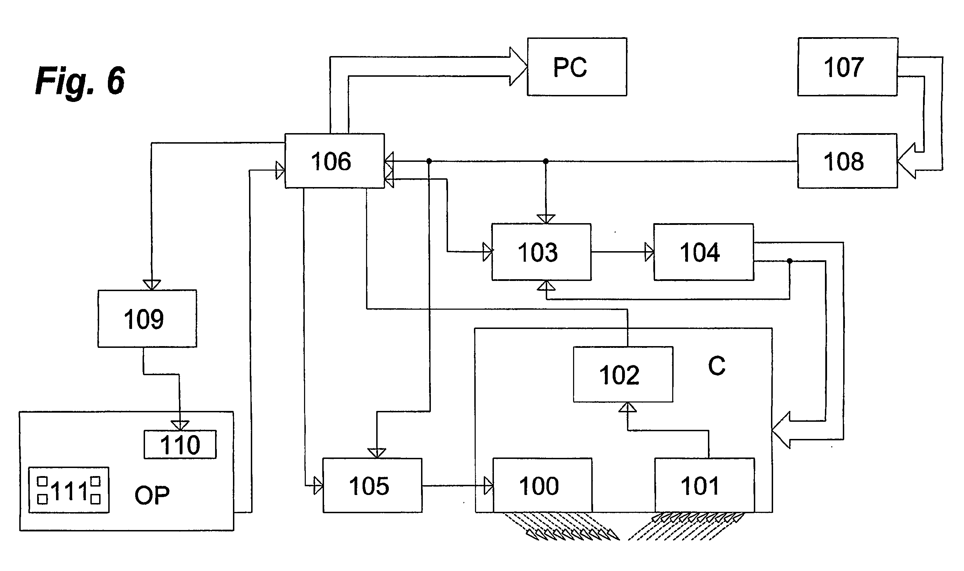

The flexible PPG pad of Figs. 2 and 3 is manufactured in the following way. A cast form for the pad body is made from wood. The cast material, a two-component silicon rubber, Rhodorsil RTV 1556 A and B (Sikema, Sweden; hardness upon curing: 28±3 Shore A) and black pigment, Intensive Black 886,(Uterm, Sweden) is mixed at room temperature in a plastic container, and the mixture is poured into the form. Incorporating black or other pigment light reduces interference by light from outside. Air bubbles are removed by placing the form in a steel container, which is evacuated. The polymer is hardened in an oven. The pad body 60 thus produced comprises windows that mirror the LED/PD pattern of the pad 1. The LED's and the PD's are accordingly put into the respective windows and fixed by gluing with transparent silicone glue, Glass Silicone Transparent (Dana, Denmark). In an additional window the probe AMP board 102 is placed and fixed by gluing. The photo diodes are connected to the probe AMP board 102 by electrical leads disposed on the rear side of the body 60. The output lead 8 from the AMP board 102 and the LED power supply lead 7 are inserted into a shielded cable 63, STC-36T-12 (Vishay Measurement Group, Germany). A cable connector, HR10A (ELFA, Sweden) is connected to free end of the shielded cable for connection to a PPG board 106 (Fig. 6). After control of optical and electrical functions, a sheet of silicone rubber 59 is glued to the rear side of the body 60 to protect the components (PDs, LEDs, probe AMP board, electrical conductors). A thin sheet of transparent silicone rubber 58 is glued to the front side of the pad so as to cover it completely. The flexible PPG pad 1 thus manufactured is about 15 mm thick. In Fig. 3 its thickness is greatly exaggerated for reasons of clarity. The same is true for the inflatable cuff 57, the lumen of which is indicated by reference number 57. If desired, the flexible PPD pad 1 may be glued or otherwise fixed to the cuff 51 at the inside thereof. Suitable LEDs are, for instance, Siemens (Germany) SFH 420 (880 nm). Suitable photodetectors are, for instance, Siemens (Germany) BPW34. A suitable cuff for blood pressure measurements can be obtained from OMRON Healthcare UK (Henfield, West Sussex, UK).

Fig. 4 illustrates the disposition of the flexible PPG pad of Figs. 2 and 3 in relation to an inflatable cuff 51 of about rectangular shape having a proximal (top) edge 53, a distal (bottom) edge 54, and left 56 and right 55 edges. The cuff 51 may be a state-of-the-art cuff used for blood pressure measurements with an inflatable portion 51 and a non-inflatable flap portion 52 provided with a fixation means (not shown) such as Velcro® tape. The PPG pad 1 of the invention is disposed close to the distal edge 54 of the cuff. When inflated, the cuff 51 thus restricts arterial blood flow proximally (upstream) of the flexible PPG pad 1 but may additionally restrict blood flow at the application area of the pad.

EXAMPLE 3. System for measuring systolic ankle blood pressure.

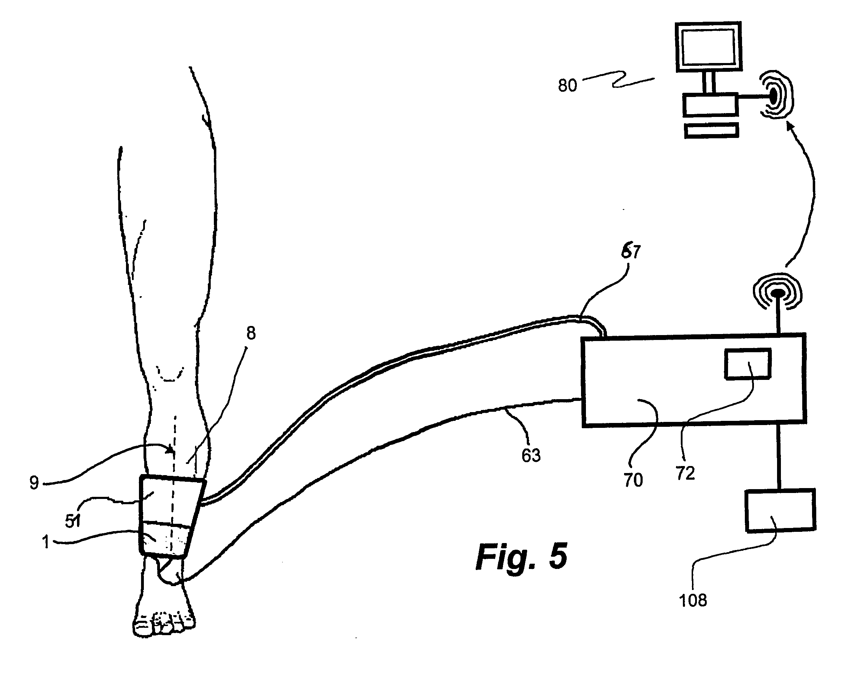

An embodiment of the system for determining systolic ankle blood pressure of the invention is schematically illustrated in Fig. 5. The system comprises a flexible PPG pad 1 of the invention and an inflatable cuff 51; both are shown mounted to the ankle of a right leg 8 in which the front edge 9 of the tibia is indicated. The flexible PPG pad 1 is positioned between the cuff 51 and the ankle and thus covered by the cuff 50. A shielded cable 63, which extends from the PPG pad 1, and a tube 67 for pressurised air, which extends from the cuff 51, are connected with a PPG/cuff pressure control unit 70, which comprises a panel 72 with a screen and operator input means. Power is supplied to the control unit 70 by a power source 108. The control unit 70 is in wireless communication with a personal computer 80 in which photodetector signal amplitude and cuff pressure data are analysed and stored.

Circuitry for assembly for measuring ankle systolic pressure.

The circuitry of the system of Fig. 5 for measuring systolic ankle blood pressure measurement is shown in greater detail in Fig. 6. It comprises at least one light source 100 and at least one photo detector 101 in a flexible PPG pad which is combined with a inflatable cuff C. The photo detector 101 is electrically connected to an AMP board 102 in the pad for amplification of the weak detector signal from which it is sent to a PPG board 106 that hierarchically controls the system. The PPG board comprises an analog to digital converter. The converted LED signal is sent to a PC in which it is analysed and stored. Via a pump board 103 the PPG board 106 controls a pump/valve assembly for inflating and deflating the cuff. The pump board 103 comprises pressure sensors the signals of which are fed to the PPG board. A safety circuit opens a magnetic valve at a selected pressure, which can be adjusted by a potentiometer. Typically the safety limit will be set at about 400 mm Hg. The PPG board 106 also controls the light source 101 via an LED driver board 105. A bedside operator panel OP comprising a display 110 and a control panel 111 connected to the PPG board 106 allows the operator to carry out the measurement. The control panel 111 comprises input means for start/reset of measurement, start/reset of cuff pressure, LED gain, etc. The LED signal and other data is presented on a display 110. The signal from the PPG board 103 fed to the display is converted in a display board 109 that also comprises latch and driver means for the display. Power from an external power supply 107 is filtered and distributed via a power board 108.

EXAMPLE 4. <u>PD signal analysis algorithm. Determination of true first systolic peak</u>. The signal analysis used to extract the local PPG peak that corresponds to the pressure in the occlusion cuff just below the systolic pressure (when arterial blood starts to pass the cuff) is performed in three steps:

a) The information reduction step is constituted by a differentiating filter (a simple first order differentiating convolution kernel), followed by a 10th order band pass Butterworth filter with cut frequencies at 4Hz and 9Hz applied to the signal in order to retain only the pulsatile features of the signal.

b) *Peak extraction step.* Extracts PPG signals corresponding to true heart beats. PPG peaks that correspond to true beats are determined by analysis of the amplitude of the signal appearing within intervals determined by, in part, the assumption that a reasonable heart rate at rest does not exceed 100 beats per min and, in part, by the most frequently occurring amplitude peak distance, i.e., the heart rate. The amplitude of the signal is determined by using a 16th order Hilpert transformer filter.

The Hilbert transformer is a filter that approximates the discrete Hilbert transform

$$f_{Hi}(x) = \frac{1}{\pi} \int_{-\infty}^{\infty} \frac{f(\tau)}{\tau - x} d\tau \,,$$

of a signal

$$\dot{f}(x)$$

which is an integral part of the analytic signal

The analytical signal is in turn used to derive the amplitude of the signal

$$\dot{f}(x)$$

as the instantaneous amplitude of

$$f(x) \text{ is } \left| f_A(\dot{x}) \right| \ \left(= \sqrt{\left[f(x)\right]^2 + \left[f_{Hi}(x)\right]^2}\right).$$

c) *Peak selection step.* Selects the peak that corresponds to the systolic pressure. The PPG peak is selected that exhibits the greatest difference in amplitude compared to the peak next in line, and which is followed by a predetermined number of peaks, typically 15-20, depending on sampling time.

It is, of course, also possible to visually determine the ankle systolic pressure by identifying in the PD v. cuff pressure trace the first peak in a row of peaks of increasing amplitude and about equal spacing.

Another method for identifying the first true peak in a PD/cuff pressure diagram is by gating. The peak with the largest or second largest amplitude (reference peak) is identified. Peaks smaller than a set fraction of the reference peak are disregarded from. The first peak having an amplitude equal or higher than said fraction is considered to be the first true peak. Gating can be used as such or in combination with other methods.

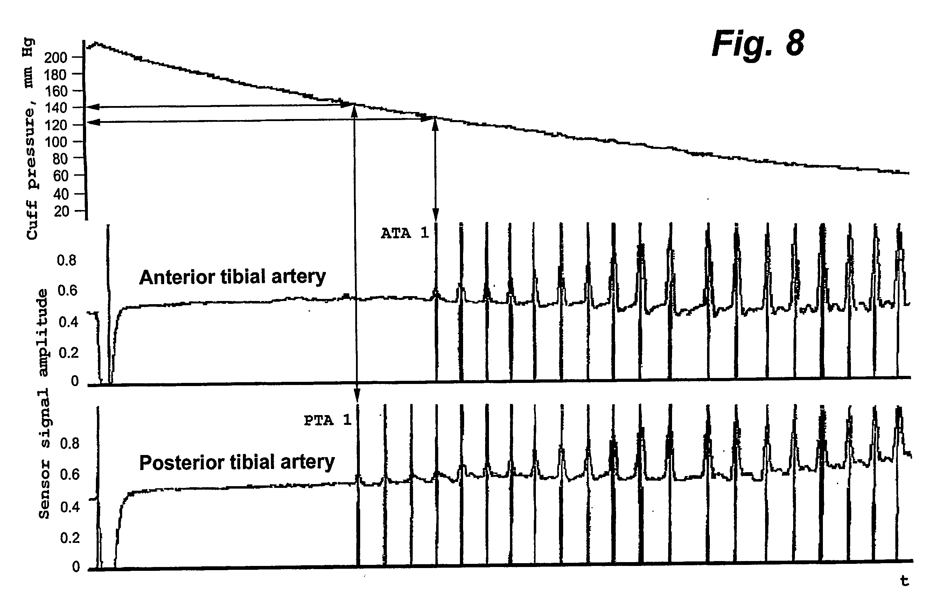

EXAMPLE 5. <u>Ankle systolic blood pressure measurement procedure</u>. The PPG pad 1 of the invention is positioned at the ankle with the marker corresponding to line 5 in line with the palpable front edge MA of the tibia. The cuff 51 is then applied on the pad in a manner that the lower edge of the cuff 51 superimposes the lower edge of the pad 1. The person on which the measurement is performed is placed in a supine position on a bed. The measurement is started by inflating the cuff 51 while monitoring the PD signals. The cuff is inflated above the pressure range at which the PD signals become essentially flat, that is, at which blood ceases to flow in the anterior ATA and posterior PTA tibial arteries. Monitoring is continued during the entire procedure of inflation and deflation of the cuff 51, which lasts about 120 seconds (cuff deflation rate approx. 3 mm Hg/s). On release of the pressure in the cuff the signals from the anterior and posterior tibial arteries, respectively, appear consecutively (Fig. 8) or about at the same time, depending on the condition of the particular patient.

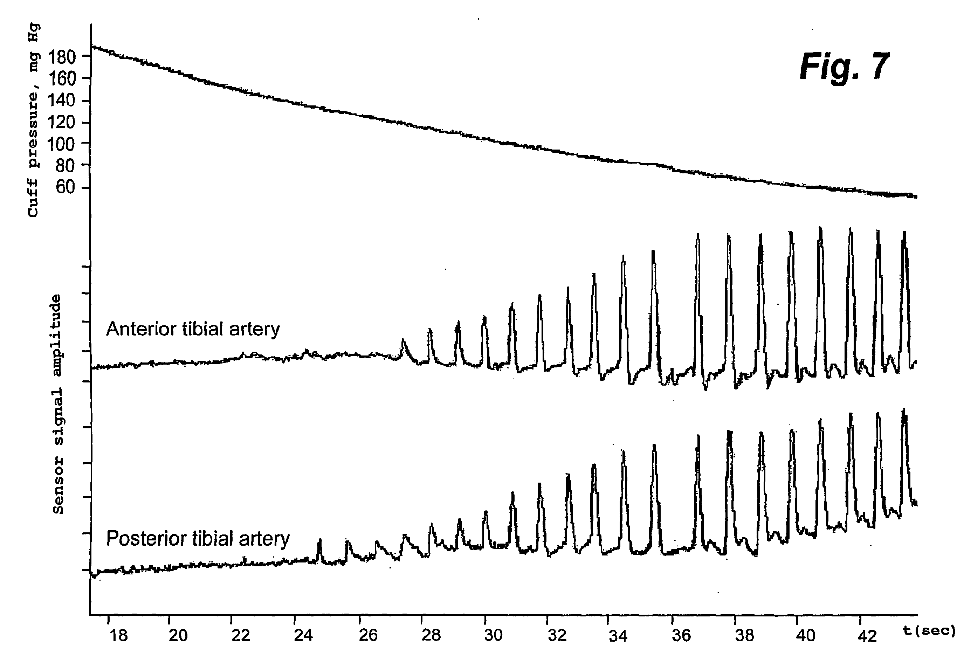

The raw PD amplitude signals from the anterior and posterior tibial arteries, respectively, are shown in Fig. 7 in relation to cuff pressure during deflation. The corresponding PC-analysed signals are shown in Fig. 8, in which also the peak corresponding to the first true pulse (heart beat) is identified in each trace. The cuff pressure for this peak corresponds to the ankle systolic pressure for the anterior and posterior tibial artery, respectively.

EXAMPLE 6. PPD pad systolic ankle blood pressure measurement evaluation - Non-invasive measurements. For comparison with the standard Doppler technique, 20 healthy volunteers (24 - 55 of age, 15 men) were examined. All subjects were placed supine on a bed at room temperature. The measurements were performed at the right ankle after five minutes of rest. Along with the PPG recordings, CW Doppler pressure measurements in the *arteria dorsalis pedis* and posterior tibial arteries were performed simultaneously using an 8-MHz handheld Doppler (MD-8, Sonotech GMBH, Schwaben, Germany). The systolic pressure was read on the aneroid sphygmomanometer at the closest 2mm Hg. Four consecutive measurements were performed, two in each ankle artery. The PPG signals were stored on the PC hard disc for later analysis. During the procedure, the PPG-derived pressures were not available to the examiner. The influence of incorrect probe positioning was tested in 18 of the subjects. Measurements were made with the cuff and pad rotated 2 cm medially and laterally from the correct position, respectively. Two measurements were performed in each position, one in each ankle artery, using the same procedure as above. A PPD pad with the LED/photo detector configuration of Figs. 2 and 3 was used.

The simultaneous CW Doppler measurements were carried out with an aneroid sphygmomanometer (Maxi-Stabil 3, Welch Allyn, New York, USA) connected to the pressure regulator unit.

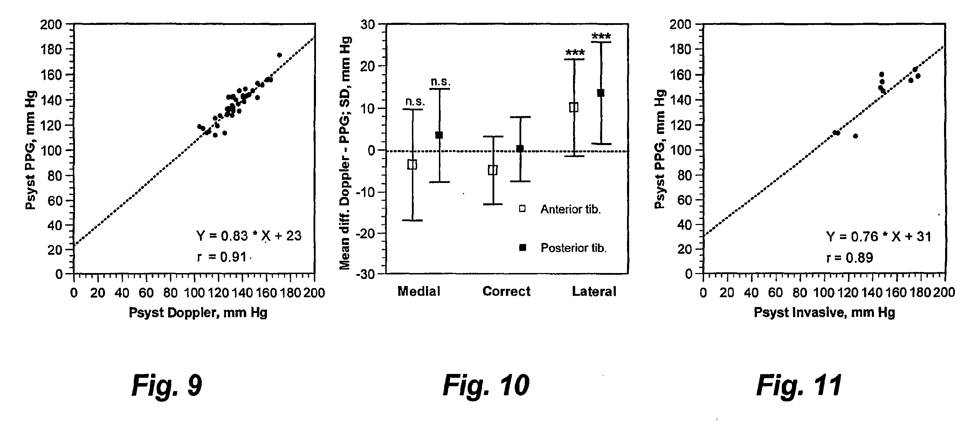

Visual analysis of the reappearance of PPG pulsations during cuff exsufflation was possible in all 80 measurements, despite numerous movement artefacts and/or noise. The results are shown in Fig. 9 (mean of two measurements in each position). Rotating the combination of cuff and pad two cm laterally from the central, predetermined "correct" position produced a mean difference between CW Doppler and PPG results in the anterior tibial artery of 10.1 mm Hg (SD 11.5). This compared to -4.9 mm Hg (SD 8.4) with the "correct" position (n=17 (one measurement excluded due to technical errors), p<0.001). In the posterior tibial artery, the mean differences were 13.6 mm Hg (SD 13.1) and 0.2 mm Hg (SD 7.6) respectively (n=18, p<0.001). A 2 cm medial rotation did not alter the mean error significantly, but the standard deviations increased (mean differences CW Doppler - PPG -3.6 (SD 13.3) and 3.4 (SD 11.1) mm Hg for the anterior and posterior arteries, respectively) (Fig. 10).

EXAMPLE 7. PPG pad ankle systolic pressure measurement evaluation - invasive measurements. Ten neurosurgical or thoracic patients at the intensive care unit (40 - 75 years old, three males) with an arterial line put in for clinical monitoring purposes were included. None had a diagnosis of peripheral arterial disease; all had palpable foot pulses. A 1.1 x 45 mm cannula (BD, UK) was inserted in the *arteria dorsalis pedis* on the *dorsum pedis.* The cannula was fixed and connected to tubings that included devices for calibration against heart level, and a damping device. The line was connected to a pressure-monitoring device (HP M 1006A/B pressure module and HP Viridia monitor, Hewlett Packard, USA). The pressure curves were carefully checked for artefacts (flushing technique), and a damping device was used when necessary to avoid under-damping ("ringing"). For practical reasons, PPG recordings were performed in the contralateral leg. Doppler systolic pressures in the *arteria dorsalis pedis* arteries of right and left leg were examined. Only subjects with a pressure difference not exceeding 5 mm Hg between legs were accepted. The intra-arterial systolic pressure from the arterial line was recorded during exsufflaton of the cuff. A minimum of six measurements were performed in each patient. In comparison with systolic ankle pressure measured intra-arterially in the *arteria dorsalis pedis,* PPG (by visual examination) underestimated systolic pressure in the contralateral anterior tibial artery by 4.5 mm Hg (SD 11.2), (Fig. 11).

*Statistics.* Continuous data are presented as their means and standard deviations (SD). 95% confidence intervals were calculated for mean differences. Pearson correlation coefficient (r) were calculated where appropriate. Comparisons were performed using Student's t-test for paired samples. P-values of less than 5% were considered statistically significant. All calculations were performed using SPSS for Windows version 11.0 (SPSS Inc., USA).

EXAMPLE 8. Simultanous measurement of ankle and arm systolic blood pressure. Ankle systolic pressure measurements with the pad of the invention were carried out in combination with the simultaneous measurement of systolic blood pressure in the arm to obtain the so-called Ankle Brachial Index (ABI). An ABI of >0.95 indicates a healthy state. Ankle pad measurements of the invention substituted the standard Doppler ultrasound technique. A pad of a design similar to that of the ankle pad of the invention but simplified, there being no need to discern between pressures in various brachial arteries, can be used for brachial systolic blood pressure measurement. The measurement of ABI can be accomplished with both pads connected to a single control unit corresponding to control unit 70 of Fig. 5. The computer 80 compares the measured ankle and brachial pressures for calculation of ABI.

**EP 2 112 902 B1**

**Claims**

1. A photoplethysmographic flexible pad (1) for measuring systolic ankle blood pressure wherein the pad is attachable to the ankle by means of an inflatable cuff, and the pad comprises two or more pairs of light emitting diodes and photo detectors, said pairs being disposed in parallel, the detectors being adapted for detecting light emitted by the respective diode into tissue and reflected from there, the pad further comprising conductor means (63) for providing power to the light emitting diodes from a power source and for putting the detectors in communication with electronic equipment for detector signal analysis, wherein the pad is of a length allowing it to extend around at least half of the circumference of the ankle of an adult person, and wherein the disposition of the light emitting diodes and the photodetectors allows separate detection of light reflected from the anterior tibial artery and from the posterior tibial artery.

2. The pad of claim 1 comprising a means for detector signal amplification.

3. The pad of claim 2, wherein the detector signal amplification means comprises an AMP board (102).

4. The pad of any of claims 1 to 3 of about rectangular shape, its short sides being designed to extend in a proximal/distal direction when mounted on the leg of a patient.

5. The pad of any of claims 1 or 4 comprising two sections of about rectangular shape, the line of division (5) being parallel to the short sides and configured for disposition along the margo anterior tibiae in a mounted position.

6. The pad of claim 5, wherein each section comprises one or more pairs of light emitting diodes and one or more pairs of photodetectors.

7. The pad of claim 1, wherein the distance between the light emitting diode and the photodetector in a pair is smaller than the distance between light emitting diodes and photodetectors in different pairs.

8. The pad of claim 1 or 6, wherein said disposition includes that of the free ends of optical fibers extending from said light emitting diodes and/or photodetectors.

9. The pad of any of claims 1 to 8, wherein the light emitting diodes and the photo detectors and/or the corresponding free ends of the optical fibers are disposed in rectangular pattern or a rectangular network pattern.

10. The pad of any of claims 1 to 9, comprising six pairs of light emitting diodes and photodetectors.

11. The combination of the pad of any of claims 1 to 10 and an inflatable ankle cuff (51).

12. The combination of claim 11, wherein the pad is fixed to the cuff.

13. The combination of claim 12, wherein the pad is releasingly fixed to the cuff.

14. A system for measuring systolic blood pressure in the ankle of a subject, comprising the measuring pad of any of claims 1 to 10, an ankle cuff fixed to the pad or separate of it, pump means for inflating and deflating the cuff, gauge means for recording the pressure in the cuff, electronic means for amplification of photodetector signals, computing means for analysing the amplified signal, and power supply means.

15. A system for determining the Ankle Brachial Index (ABI) of a subject, comprising the system of claim 14, means configured for determining brachial systolic blood pressure, and means configured for calculating ABI.

16. The system of claim 15, wherein the means for determining brachial systolic pressure include a brachial measuring pad comprising at least one pair of light emitting diode and photodetector, a brachial cuff, pump means for inflating and deflating the brachial cuff, gauge means for recording the pressure in the brachial cuff, electronic means for amplification of photodetector signals from the brachial measuring pad, and computing means configured for analysing the amplified signal from the brachial cuff.

9

**Patentansprüche**

1.  Photoplethysmographisches flexibles Pad (1) zum Messen des systolischen Blutdrucks am Knöchel, wobei das Pad mittels einer aufblasbaren Manschette am Knöchel anbringbar ist und das Pad zwei oder mehr Paare von Leuchtdioden und lichtempfindlichen Sensoren umfasst, wobei die Paare parallel angeordnet sind, wobei die Sensoren so ausgelegt sind, dass sie Licht detektieren, das von der jeweiligen Diode in Gewebe abgestrahlt und von dort reflektiert wird, wobei das Pad ferner Leitermittel (63) umfasst, um die Leuchtdioden von einer Stromquelle aus mit Strom zu versorgen und um die Sensoren mit elektronischer Ausrüstung für die Sensorsignalanalyse in Verbindung zu bringen, wobei das Pad eine Länge aufweist, die ermöglicht, dass es um zumindest den halben Umfang des Knöchels einer erwachsenen Person verläuft, und wobei die Anordnung der Leuchtdioden und der lichtempfindlichen Sensoren die separate Detektion von Licht ermöglicht, das von der vorderen Schienbeinarterie und von der hinteren Schienbeinarterie reflektiert wird.

2.  Pad nach Anspruch 1, das ein Mittel für die Sensorsignalverstärkung umfasst.

3.  Pad nach Anspruch 2, wobei das Sensorsignalverstärkungsmittel eine Verstärkerplatine (102) umfasst.

4.  Pad nach einem der Ansprüche 1 bis 3 mit in etwa rechteckiger Form, wobei die kurzen Seiten so ausgestaltet sind, dass sie in einer proximalen/distalen Richtung verlaufen, wenn es an dem Bein eines Patienten angebracht ist.

5.  Pad nach einem der Ansprüche 1 oder 4, das zwei Abschnitte mit in etwa rechteckiger Form umfasst, wobei die Teilungslinie (5) parallel zu den kurzen Seiten verläuft und so eingerichtet ist, dass sie in einer angebrachten Stellung entlang dem Margo anterior des Schienbeins angeordnet ist.

6.  Pad nach Anspruch 5, wobei jeder Abschnitt ein oder mehrere Paare von Leuchtdioden und ein oder mehrere Paare von lichtempfindlichen Sensoren umfasst.

7.  Pad nach Anspruch 1, wobei der Abstand zwischen der Leuchtdiode und dem lichtempfindlichen Sensor in einem Paar geringer ist als der Abstand zwischen Leuchtdioden und lichtempfindlichen Sensoren in unterschiedlichen Paaren.

8.  Pad nach Anspruch 1 oder 6, wobei die Anordnung beinhaltet, dass die freien Enden von Lichtwellenleitern von den Leuchtdioden und/oder lichtempfindlichen Sensoren aus verlaufen.

9.  Pad nach einem der Ansprüche 1 bis 8, wobei die Leuchtdioden und die lichtempfindlichen Sensoren und/oder die entsprechenden freien Enden der Lichtwellenleiter in einem rechteckigen Muster oder einem rechteckigen Netzmuster angeordnet sind.

10. Pad nach einem der Ansprüche 1 bis 9, das sechs Paare von Leuchtdioden und lichtempfindlichen Sensoren umfasst.

11. Kombination aus dem Pad nach einem der Ansprüche 1 bis 10 und einer aufblasbaren Knöchelmanschette (51).

12. Kombination nach Anspruch 11, wobei das Pad an der Manschette befestigt ist.

13. Kombination nach Anspruch 12, wobei das Pad lösbar an der Manschette befestigt ist.

14. System zum Messen des systolischen Blutdrucks im Knöchel eines Patienten, das das Messpad nach einem der Ansprüche 1 bis 10, eine Knöchelmanschette, die an dem Pad befestigt oder separat davon ist, Pumpmittel zum Aufblasen und Ablassen der Luft aus der Manschette, Messmittel zum Aufzeichnen des Drucks in der Manschette, elektronische Mittel zum Verstärken von Signalen lichtempfindlicher Sensoren, Rechenmittel zum Analysieren des verstärkten Signals sowie Stromversorgungsmittel umfasst.

15. System zum Ermitteln des Knöchel-Arm-Index (ABI) eines Patienten, das das System von Anspruch 14, Mittel, die so eingerichtet sind, dass sie den systolischen Blutdruck am Oberarm bestimmen, und Mittel umfasst, die so eingerichtet sind, dass sie den ABI berechnen.

16. System nach Anspruch 15, wobei die Mittel zum Bestimmen des systolischen Drucks am Oberarm ein Oberarmmesspad, das mindestens ein Paar aus Leuchtdiode und lichtempfindlichem Sensor umfasst, eine Oberarmmanschette, Pumpmittel zum Aufblasen und Ablassen der Luft aus der Oberarmmanschette, Messmittel zum Aufzeichnen des Drucks in der Oberarmmanschette, elektronische Mittel zum Verstärken von Signalen lichtempfindlicher Sensoren von dem Oberarmmesspad und Rechenmittel aufweisen, die so eingerichtet sind, dass sie das verstärkte Signal von der Oberarmmanschette analysieren.

## Revendications

1. Élément flexible (1) photopléthysmographique destiné à mesurer la pression sanguine systolique dans la cheville, dans lequel l'élément peut être relié à la cheville au moyen d'un brassard gonflable, et l'élément comprend deux paires de diodes électroluminescentes et photodétecteurs ou plus, lesdites paires étant disposées en parallèle, les détecteurs étant adaptés pour détecter de la lumière émise par la diode respective dans le tissu et réfléchie depuis celui-ci, l'élément comprenant en outre des moyens conducteurs (63) pour fournir de l'électricité aux diodes électroluminescentes depuis une source électrique et pour mettre les détecteurs en liaison avec un équipement électronique pour l'analyse de signaux de détecteur, dans lequel l'élément présente une longueur lui permettant de s'étendre autour d'au moins la moitié de la circonférence de la cheville d'une personne adulte, et dans lequel la disposition des diodes électroluminescentes et des photodétecteurs permet une détection séparée de la lumière réfléchie par l'artère tibiale antérieure et par l'artère tibiale postérieure.

2. Élément selon la revendication 1, comprenant un moyen destiné à l'amplification du signal de détecteur.

3. Élément selon la revendication 2, dans lequel le moyen d'amplification du signal de détecteur comprend une carte d'amplification (102).

4. Élément selon l'une quelconque des revendications 1 à 3 de forme approximativement rectangulaire, ses petits côtés étant conçus pour s'étendre dans une direction proximale/distale quand il est monté sur la jambe d'un patient.

5. Élément selon l'une quelconque des revendications 1 ou 4, comprenant deux parties de forme approximativement rectangulaire, la ligne de division (5) étant parallèle aux petits côtés et configurée pour la disposition le long du margo anterior du tibia dans une position montée.

6. Élément selon la revendication 5, dans lequel chaque partie comprend une ou plusieurs paires de diodes électroluminescentes et une ou plusieurs paires de photodétecteurs.

7. Élément selon la revendication 1, dans lequel la distance entre la diode électroluminescente et le photodétecteur dans une paire est inférieure à la distance entre des diodes électroluminescentes et des photodétecteurs dans des paires différentes.

8. Élément selon la revendication 1 ou 6, dans lequel ladite disposition comprend celle des extrémités libres de fibres optiques s'étendant depuis lesdites diodes électroluminescentes et/ou lesdits photodétecteurs.

9. Élément selon l'une quelconque des revendications 1 à 8, dans lequel les diodes électroluminescentes et les photodétecteurs et/ou les extrémités libres correspondantes des fibres optiques sont disposés dans une configuration rectangulaire ou une configuration de réseau rectangulaire.

10. Élément selon l'une quelconque des revendications 1 à 9, comprenant six paires de diodes électroluminescentes et photodétecteurs.

11. Combinaison de l'élément selon l'une quelconque des revendications 1 à 10 et d'un brassard de cheville gonflable (151).

12. Combinaison selon la revendication 11, dans laquelle l'élément est fixé au brassard.

13. Combinaison selon la revendication 12, dans laquelle l'élément est fixé de manière détachable au brassard.

14. Système destiné à mesurer la pression sanguine systolique dans la cheville d'un sujet, comprenant l'élément de mesure selon l'une quelconque des revendications 1 à 10, un brassard de cheville fixé à l'élément ou séparé de celui-ci, des moyens de pompe destinés à gonfler et dégonfler le brassard, des moyens de mesure pour enregistrer la pression dans le brassard, des moyens électroniques pour l'amplification de signaux de photodétecteur, des moyens informatiques pour analyser le signal amplifié, et des moyens d'alimentation électrique.

15. Système destiné à déterminer l'index de pression systolique (IPS) d'un sujet, comprenant le système selon la revendication 14, des moyens configurés pour déterminer la pression sanguine systolique humérale et des moyens configurés pour calculer l'IPS.

16. Système selon la revendication 15, dans lequel les moyens pour déterminer la pression systolique humérale comportent un élément de mesure huméral comprenant au moins une paire de diode électroluminescente et photodétecteur, un brassard huméral, des moyens de pompe pour gonfler et dégonfler le brassard huméral, des moyens de mesure pour enregistrer la pression dans le brassard huméral, des moyens électroniques pour l'amplification de signaux de photodétecteur de l'élément de mesure huméral, et des moyens informatiques configurés pour analyser le signal amplifié du brassard huméral.

**Fig. 1**

**Fig. 2**

*Fig. 3*

Fig. 4

**Fig. 5**

EP 2 112 902 B1

**Fig. 6**

EP 2 112 902 B1

**Fig. 7**

Fig. 8

Cuff pressure, mm Hg

200 180 160 140 120 100 80 60 40 20

ATA 1

Anterior tibial artery

PTA 1

Posterior tibial artery

Sensor signal amplitude

0.8 0.6 0.4 0.2 0

0.8 0.6 0.4 0.2 0

t

**Fig. 9**

**Fig. 10**

**Fig. 11**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6120459 A
- US 6533729 B1

**Non-patent literature cited in the description**

- **E O'BRIEN et al.** *Brit. Med. J.,* vol. 322, 531-536
- **J STOFFERS et al.** *Scand. J. Prim. Health Care,* 1991, vol. 9, 109-114
- **K W JOHNSTON et al.** *J. Vasc. Surg.,* 1987, vol. 6, 147-151
- **F G R FOWKES et al.** *J. Epidem. Comm. Health,* 1988, vol. 42, 128-133
- **S A RAY et al.** *Br. J. Surg.,* 1994, vol. 81, 188-190
- **C M FISHER et al.** *J. Vasc. Surg.,* 1996, vol. 24, 871-875
- **M ADISESHIAH et al.** *Ann. Royal Coll. Surg. Engl.,* 1987, vol. 69, 271-273
- **B Y LEE et al.** *J Vasc. Surg.,* 1996, vol. 23, 116-122
- **B JÖNSSON et al.** *Clin. Physiol.,* 2001, vol. 21 (2), 155-163
- **K YAMAKOSHI et al.** *Med. Biol. Eng. Comput.,* 1982, vol. 20, 307-313
- **R CHAWLA et al.** *Anesth. Analg.,* 1992, vol. 74, 196-200
- **M LANGBAUM et al.** *J. Pediatr.,* 1994, vol. 125, 591-595
- **S. TANAKA ; K. YAMAKOSHI.** *Med. & Biol. Eng. & Comput.,* 1996, vol. 34, 441-447